# EUROPÄISCHE PATENTSCHRIFT

(11) **EP 1 673 446 B1**
(45) Veröffentlichungstag und Bekanntmachung des Hinweises auf die Patenterteilung: **17.12.2008**
(21) Anmeldenummer: 04790192.1
(22) Anmeldetag: 08.10.2004
(51) Int. Cl.: C12N 5/08, A61L 27/00

(54) **KNORPELERSATZIMPLANTAT UND VERFAHREN ZUR HERSTELLUNG EINES KNORPELERSATZIMPLANTATS**
CARTILAGE REPLACEMENT IMPLANT AND METHOD FOR PRODUCING A CARTILAGE REPLACEMENT IMPLANT
PROTHESE DE CARTILAGE ET PROCEDE POUR REALISER UNE PROTHESE DE CARTILAGE

(30) Priorität: 13.10.2003 DE 10348219
(43) Veröffentlichungstag der Anmeldung: 28.06.2006
(73) Patentinhaber: Aesculap AG, 78532 Tuttlingen (DE)
(72) Erfinder: FRITZ, Jürgen, 72144 Dusslingen (DE); GAISSMAIER, Christoph, 72070 Tübingen (DE); AICHER, Wilhelm, Karl, 72119 Ammerbuch (DE)
(74) Vertreter: Hoeger, Stellrecht & Partner Patentanwälte
(86) Internationale Anmeldenummer: PCT/EP2004/011240
(87) Internationale Veröffentlichungsnummer: WO 2005/038016

(56) Entgegenhaltungen:
- EP-A- 1 273 312
- US-A- 6 080 194
- CHERUBINO P ET AL: "Autologous chondrocyte implantation using a bilayer collagen membrane: a preliminary report." JOURNAL OF ORTHOPAEDIC SURGERY (HONG KONG) JUN 2003, Bd. 11, Nr. 1, Juni 2003 (2003-06), Seiten 10-15, XP002310328 ISSN: 1022-5536 & Gefunden im Internet: URL:http://www.verigen.de/index.php_lang=d e&pg=0&pug=0&hs=1&hus=0>
- CUI Y L ET AL: "Biomimetic surface modification of poly(l-lactic acid) with chitosan and its effects on articular chondrocytes in vitro" BIOMATERIALS, ELSEVIER SCIENCE PUBLISHERS BV., BARKING, GB, Bd. 24, Nr. 21, September 2003 (2003-09), Seiten 3859-3868, XP004431167 ISSN: 0142-9612
- MA ZUWEI ET AL: "Immobilization of natural macromolecules on poly-L-lactic acid membrane surface in order to improve its cytocompatibility." JOURNAL OF BIOMEDICAL MATERIALS RESEARCH. 2002, Bd. 63, Nr. 6, 2002, Seiten 838-847, XP002310329 ISSN: 0021-9304
- SFA-ARTHROSKOPIE AKTUELL, Bd. 16, 2. Oktober 2003 (2003-10-02), Seiten 4-14, XP009041180

## Beschreibung

Die Erfindung betrifft ein Knorpelersatzimplantat zur biologischen Regeneration eines geschädigten Knorpelbereichs eines Gelenkknorpels eines menschlichen Körpers, umfassend einen eine Defektanlagefläche zur Anlage an den geschädigten Knorpelbereich aufweisenden Zellträger, welcher derart ausgebildet und aufgebaut ist, daß er mit menschlichen Zellen besiedelt werden kann, wobei der Zellträger mit einer von der Defektanlagefläche weg weisenden Zellträgerfläche flächig an einem Träger anliegt und mit dem Träger verbunden ist.

Ferner betrifft die Erfindung ein Verfahren zum Herstellen eines Knorpelersatzimplantats zur biologischen Regeneration eines geschädigten Gelenkknorpels eines menschlichen Körpers, wobei ein eine Defektanlagefläche zur Anlage an den geschädigten Knorpelbereich aufweisender Zellträger verwendet wird, welcher derart ausgebildet und aufgebaut ist, daß er mit menschlichen Zellen besiedelt werden kann, wobei der Zellträger mit einer von der Defektanlagefläche weg weisenden Zellträgerfläche flächig mit einem Träger verbunden wird.

Knorpelersatzimplantate der eingangs beschriebenen Art werden ohne oder nach vorheriger Impfung mit körpereigenen Zellen zur Rekonstruktion von Knorpeldefekten an Gelenkknorpeln des menschlichen Körpers eingesetzt. Üblicherweise werden Biomaterialien ausgewählt, welche vom Körper resorbiert werden können. Allerdings besteht bei Biomaterialien für eine zellfreie Implantation mit oder ohne Wachstumsfaktoren oder bei der trägergekoppelten Transplantation von Zellen, beispielsweise aus körpereigenen Zellen gewonnene und vermehrte Chondrocyten oder mesenchymale Stammzellen, in einen Gewebedefekt, beispielsweise einen geschädigten Knorpelbereich, das Problem, daß die verwendeten Biomaterialien im Verlauf ihrer Resorption die Neigung entwickeln, zu kontrahieren. Eine solche Kontraktion und damit Schrumpfung von Biomaterialien kann häufig nach ihrem Kontakt mit Zellen sowohl in vitro als auch in vivo beobachtet werden und wird vor allem durch kontraktile Elemente eingesäter beziehungsweise eingewanderter Zellen hervorgerufen. Eine unerwünschte Folge hiervon ist, daß bei beginnender Resorption der Biomaterialien die mechanische Stabilität der Implantatstrukturen nachläßt und die zellvermittelte Kontraktion zu einer erheblichen Formän derung und Volumenkontraktion des Biomaterials führt.

Bei der biologischen Regeneration unterschiedlicher Gewebe des Bewegungsapparats und hier insbesondere bei der Rekonstruktion von druckbelasteten Strukturen, wie beispielsweise dem Gelenkknorpel im Knie, dem Anulus fibrosus der Bandscheibe oder dem Nukleus Pulposus der Bandscheibe, ist es jedoch von großer Bedeutung, daß es zu einer möglichst spaltfreien Fusion zwischen dem aus dem Ersatzimplantat entstehenden Regenerat und den gesunden Umgebungsstrukturen des Empfängerlagers, also des defekten Gewebebereichs, kommt. Spaltbildungen zwischen dem Regenerat und gesunden Umgebungsstrukturen des Empfängerlagers, oder mit anderen Worten, das ausbleibende stabile Verwachsen von Implantat und Empfängerstrukturen, können jedoch das funktionale Resultat der biologischen Rekonstruktion im weiteren Verlauf gefährden. Beispielsweise stellt eine Spaltbildung im Übergangsbereich zwischen ortsständigem Knorpel und Ersatzimplantat auf Knorpelniveau, eine biomechanische Schwachstelle dar und bildet häufig den Ausgangspunkt weiterer Knorpeldegenerationen.

In der US 6,080,194 sowie in CHERUBINO P ET AL: "Autologous chondrocyte implantation using a bilayer collagen membrane: a preliminary report." JOURNAL OF ORTHOPAEDIC SURGERY (HONG KONG) JUN 2003, Bd. 11, Nr. 1, Juni 2003 (2003-06), Seiten 10 - 15, XP002310328 ISSN: 1022-5536 ist die matrixgekoppelte autologe Chondrocyten Implantation unter Verwendung einer Kollagen I/III-Membran mit Bilayer-Struktur beschrieben. In der EP 1 273 312 A2 sowie in CUI Y L ET AL:"Biomimetic surface modification of poly(1-lactic-acid) with chitosan and its effects on articular chondrocytes in vitro "BIOMATERIALS, ELSEVIER SCIENCE PUBLISHERS BV., BARKING, GB, Bd. 24, Nr. 21, September 2003 (2003-09), Seiten 3859-3868, XP 004431167 ISSN: 0142-9612 und MA ZUWEI ET AL: "Immobilization of natural macromolecules on poly-L-lactic acid membrane surface in order to improve its cytocompatibility." JOURNAL OF BIOMEDICAL MATERIALS RESEARCH. 2002, Bd. 63, Nr. 6, 2002, Seiten 838-847, XP 002310329 ISSN: 0021-9304 ist die Verwendung von Membranen bestehend aus einem synthetischen Polymer (PLGA, PLLA) beschichtet mit Kollagen oder Chitosan oder Gelatine zur Rekonstruktion von Knorpeldefekten an Knorpeln beschrieben.

Es ist daher Aufgabe der vorliegenden Erfindung, ein Knorpelersatzimplantat und ein Verfahren zum Herstellen eines Knorpelersatzimplantats so zu verbessern, daß eine Spaltbildung nach Implantation des Knorpelersatzimplantats zwischen angrenzenden Kontaktflächen des Implantats und umgebendem Empfängergewebe minimiert wird.

Diese Aufgabe wird bei einem Knorpelersatzimplantat der eingangs beschriebenen Art erfindungsgemäß dadurch gelöst, daß der Zellträger mindestens zwei voneinander unabhängige Zellträgerelemente umfasst und daß jedes der mindestens zwei Zellträgerelemente mit dem Träger verbunden ist.

Der Zellträger, welcher vor oder nach der Implantation mit Zellen beimpft werden kann, wird in gewünschter Weise vom Körper resorbiert werden, wobei er jedoch in bekannter Weise kontrahiert. Eine Kontraktion des gesamten Knorpelersatzimplantats läßt sich jedoch durch Verwendung des Trägers verhindern, welcher seine Form und Struktur länger beibehalten kann als der Zellträger. Dadurch wird eine Spaltbildung zwischen dem Knorpelersatzimplantat und umliegendem, ungeschädigtem Knorpelgewebe verhindert oder zumindest minimiert. Auf diese Weise werden biomechanische Schwachstellen im Übergangsbereich zwischen Implantat und verbliebenen Körpergewebe vermieden. Bei der Resorption des Zellträgers können Zugkräfte entstehen, die im Träger zum Aufbau von Druckkräften führen, so daß eine wellige Verwerfung des gesamten Implantats eintreten kann. Dadurch kann die Gefahr einer Spaltbildung an den Rändern des Implantats erhöht werden. Vorteilhaft ist es daher, daß der Träger mindestens zwei voneinander unabhängige Zellträgerelemente umfaßt und daß jedes der mindestens zwei Zellträgerelemente mit dem Träger verbunden ist. Eine derartige Aufteilung des einen Unterbau des Implantats bildenden Zellträgers in Zellträgerelemente oder Parzellen, welche insbesondere nicht vollständig direkt miteinander verbunden sein müssen, hat zur Folge, daß nur einzelne Zellträgerelemente kontrahieren. Auf diese Weise läßt sich die kontraktionsbedingte Schrumpfung des gesamten Zellträgers als Einheit und auch des Gesamtimplantats verhindern, so daß sich die Kontaktflächen von Implantat und das Implantat nach Implantation umgebenden Empfängerstrukturen nicht voneinander weg bewegen. Es werden vielmehr einzelne Trennspalte zwischen den Zellträgerelementen durch Kontraktion der einzelnen Zellträgerelemente vergrößert. Bei einer Vielzahl von Zellträgerelementen werden somit eine Vielzahl von Trennspalten gebildet, welche gerade keine Gesamtschrumpfung des Implantats und keine große Spaltenbildung am Implantatrand zur Folge haben und jeweils für sich von untergeordneter Bedeutung sind.

Um Abstoßungsreaktion des Körpers nach Einsetzen des Knorpelersatzim plantats zu vermeiden, kann es günstig sein, wenn das Knorpelersatzimplantat aus mindestens einem körperverträglichen Material hergestellt ist.

Falls der Zellträger vor der Implantation mit beispielsweise körpereigenen Zellen beimpft wird, ist es wünschenswert, daß das Implantat nach Einwachsen der körpereigenen Zellen entfernt werden kann. Dies läßt sich in vorteilhafter Weise besonders einfach dadurch erreichen, daß das mindestens eine körperverträgliche Material resorbierbar ist.

Grundsätzlich wäre es denkbar, den Zellträger und den Träger aus identischen Materialien, insbesondere körperverträglichen Materialien, herzustellen. Sie könnten sich dann beispielsweise allein aufgrund ihrer Struktur unterscheiden. Vorteilhaft ist es jedoch, wenn der Zellträger und der Träger aus unterschiedlichen körperverträglichen Materialien hergestellt sind. Dies ermöglicht es, den Zellträger optimal auszubilden zur Aufnahme von Zellen zur Regeneration des Defekts und den Träger zur Stabilisierung des mit diesem verbundenen Zellträgers und zur Vermeidung von Spaltbildungen am Rand des Implantats.

Die Stabilität des Implantats wird insgesamt erhöht und eine Spaltbildung am Implantatrand verringert beziehungsweise ganz vermieden, wenn der Zellträger und der Träger unterschiedlich lange Resorptionszeiten aufweisen.

Vorteilhafterweise ist die Resorptionszeit des Trägers länger als die Resorptionszeit des Zellträgers. Dadurch kann die Stabilität des Implantats auch noch nach einsetzender Resorption des Zellträgers gewährleistet werden.

Vorzugsweise weisen der Zellträger und der Träger unterschiedliche Resorptionskinetiken auf. Beispielsweise kann das Implantat so aufgebaut sein, daß der Träger zunächst sehr langsam resorbiert wird und eine beschleunigte Resorption des Trägers erst nach vollständiger Resorption des Zellträgers einsetzt, welcher schneller resorbiert wird.

Um möglichst einen idealen Ersatz für einen defekten Knorpelbereich bereitstellen zu können, ist es günstig, wenn der Träger derart ausgebildet und aufgebaut ist, daß er mit menschlichen Zellen besiedelt werden kann. Auf diese Weise ist es möglich, sowohl den Zellträger als auch den Träger mit Zellen zu besiedeln, so daß ein Zellwachstum und ein Verwachsen des Implantats mit umgebendem Gewebe aus allen Teilen des Implantats heraus möglich ist.

Um eine optimale Anpassung des Implantats an einen geschädigten Knorpelbereich zu ermöglichen, kann es vorteilhaft sein, wenn der Zellträger mindestens zwei unterschiedliche Zellträgerschichten umfaßt. Diese können sich beispielsweise in der Art des verwendeten Materials oder in ihrer Struktur unterscheiden, so daß sie für eine Impfung auch mit unterschiedlichen Zellen optimal ausgebildet werden können.

Grundsätzlich ist es denkbar, ein Implantat bereitzustellen, welches einen von menschlichen Zellen freien Zellträger umfaßt. Ein solches Implantat ermöglicht es nach seiner Implantation, Stammzellen aus dem Knochenmark durch Einbluten aufzunehmen. Um jedoch ein besonders gutes Einwachsen des Implantats zu erreichen, kann der Zellträger mit menschlichen Zellen beimpft sein.

Eine optimale Rekonstruktion des geschädigten Knorpelbereich wird möglich, wenn die menschlichen Zellen aus körpereigenen Zellen gezüchtete und vermehrte Chondrocyten sind. In den Defekt werden damit Zellen desjenigen Zelltyps eingebracht, der dem benachbarten, gesunden Gewebe in seiner ausdifferenzierten Form am ähnlichsten ist. Auf diese Weise kann eine Abstoßungsreaktion durch den Körper praktisch ausgeschlossen werden.

Gemäß einer bevorzugten Ausführungsform der Erfindung kann vorgesehen sein, daß der Zellträger und der Träger eine unterschiedliche mechanische Stabilität aufweisen. So kann entweder der Zellträger oder der Träger mechanisch stabiler sein und zum Zusammenhalt des Implantats während des Einwachsens beitragen.

Günstig ist es, wenn der Träger eine höhere mechanische Stabilität als der Zellträger aufweist. Beispielsweise kann der Zellträger optimiert für die Aufnahme von Zellen ausgebildet sein, und daher vorzugsweise eine mechanisch instabilere Struktur aufweist. Der mechanisch stabilere Träger gewährleistet dann die Stabilität des Implantats insgesamt während des Einwachsens.

Grundsätzlich wäre es denkbar, daß der Zellträger und der Träger identische Elastizitätsmoduln aufweisen. Vorzugsweise weisen der Zellträger und der Träger jedoch unterschiedliche Elastizitätsmoduln auf. Dadurch können gezielt Stabilitäten des Zellträgers und des Trägers in gewünschter Weise voreingestellt werden.

Um einen besonders stabilen Träger zu erhalten, ist es günstig, wenn der Elastizitätsmodul des Trägers größer ist als der Elastizitätsmodul des Zellträgers. So wird die Form des Implantats im wesentlichen durch den inelastischeren Träger vorgegeben.

Ferner wäre es denkbar, daß der Träger und der Zellträger eine identische Struktur aufweisen, also beispielsweise auch eine gleich dichte Struktur. Vorteilhaft ist es aber, wenn der Träger eine dichtere Struktur als der Zellträger aufweist. Dadurch kann der Zellträger besonders gut Zellen aufnehmen und einlagern. Dagegen hat eine dichtere Struktur des Trägers zusätzlich den Vorteil, daß der Träger je nach Dichte des Trägers den defekten Knorpelbereich nach außen abdichten kann.

Um ein unerwünschtes Lösen des Zellträgers vom Träger zu verhindern, ist es vorteilhaft, wenn der Zellträger unlösbar mit dem Träger verbunden ist. Beispielsweise läßt sich dann das Implantat aus einer Träger/Zellträger-Struktur für die Implantation präparieren, indem es beispielsweise zurechtgeschnitten wird, wobei sich aufgrund der unlösbaren Verbindung der Zellträger nicht vom Träger lösen kann.

Besonders gut läßt sich der Zellträger mit Zellen impfen oder eignet er sich zum Einbluten von Stammzellen, wenn er eine schwammartige Struktur aufweist.

Vorzugsweise weist der Zellträger eine Zellträgerschichtdicke in einem Bereich von 0,3 mm bis 3,5 mm auf. Damit lassen sich mit dem Implantat defekte Knorpelbereiche unterschiedlicher Tiefe ausfüllen.

Günstig ist es, wenn der Zellträger eine Zellträgerschichtdicke in einem Bereich von 1,0 mm bis 3,5 mm aufweist.

Je nach Tiefe des defekten Knorpelbereichs ist es vorteilhaft, wenn der Träger eine Trägerschichtdicke in einem Bereich von 0,01 mm bis 0,8 mm aufweist. Je nach erforderlicher Stabilität und Größe des Defektbereichs können Implantate mit kleineren oder größeren Trägerschichtdicken ausgewählt werden.

Günstig ist es ferner, wenn der Träger eine Trägerschichtdicke in einem Bereich von 0,2 mm bis 0,4 mm aufweist.

Gemäß einer bevorzugten Ausführungsform der Erfindung kann vorgesehen sein, daß der Zellträger aus einem Material hergestellt ist, welches eine Porenweite in einem Bereich von 30 µm bis 500 µm aufweist. Je nach Art des Knorpeldefekts, beispielsweise in Abhängigkeit vom betroffenen Gelenk, können unterschiedliche Porenweiten gewählt werden, um Zellen in vorteilhafter Weise einzuimpfen.

Vorzugsweise ist der Zellträger aus einem Material hergestellt, welches eine Porenweite in einem Bereich von 80 µm bis 150 µm aufweist.

Günstig ist es, wenn der Träger aus einem porösen Material hergestellt ist, welches eine Porenweite von maximal 20 µm aufweist. Eine Perfusion der Zellen durch den Träger in den freien Gelenkraum ist dadurch ausgeschlossen. Ein Transport von Substanzen in der Gelenkflüssigkeit, welche zur Ernährung von in den Zellträger eingebrachten Zellen erforderlich sind, durch den Träger hindurch ist aber gewährleistet.

Um möglichst keine Verwerfungen des Implantats zu gestatten, ist es günstig, wenn die mindestens zwei Zellträgerelemente Zellträgerelementflächen aufweisen, und wenn Zellträgerelementflächen benachbarter Zellträgerelemente lose aneinander angrenzen. Dadurch ist sichergestellt, daß benachbarte Zellträgerelemente nur über den Träger miteinander verbunden sind, jedoch keine direkte Verbindung zwischen benachbarten Zellträgerelementen besteht. So wird erreicht, daß jeweils nur einzelne Zellträgerelemente schrumpfen, was zwar Spalte zwischen benachbarten Zellträgerelementen vergrößert, jedoch einen Spalt zwischen dem Implantat und das Implantat umgebendem Gewebe minimiert.

Damit der Spalt nach Resorption des Implantats möglichst klein gehalten werden kann, ist es von Vorteil, wenn die mindestens zwei Zellträgerelemente durch mindestens einen Schnittspalt getrennt sind, welcher durch Einschneiden eines einstückigen Zellträgers hergestellt ist.

Um beispielsweise gekrümmte Knorpelbereiche optimal ausfüllen zu können, kann es von Vorteil sein, wenn die mindestens zwei Zellträgerelemente durch mindestens eine Spalte getrennt sind und wenn die mindestens eine Spalte eine Breite in einem Bereich von 0,1 bis 0,8 mm aufweist. Dadurch kann das Implantat vor dem Einsetzen gekrümmt werden, wodurch eine effektive Spaltbreite zwischen benachbarten Zellträgerelementen minimiert werden kann.

Vorteilhaft ist es, wenn die mindestens eine Spalte mit einem körperverträglichen, resorbierbaren Material aufgefüllt ist. So läßt sich das Implantat in besonders kompakter Form in den Knorpelbereich einsetzen.

Um ein Verwerfen des Implantats in der oben beschriebenen Weise zu vermeiden, also das Ausbilden einer welligen Struktur nach beginnender Resorption, insbesondere des Zellträgers, ist es günstig, wenn der mindestens eine Schnittspalt oder die mindestens eine Spalte eine Tiefe aufweisen, die mindestens der halben Schichtdicke des Zellträgers entspricht.

Grundsätzlich ist eine Vielzahl unterschiedlicher körperverträglicher Materialien zur Herstellung des Implantats geeignet. Vorteilhaft ist es jedoch, wenn das mindestens eine körperverträgliche Material ein kollagenes Vlies ist.

Ferner kann es günstig sein, wenn das mindestens eine körperverträgliche Material ein Polymer ist, vorzugsweise ein synthetisches Polymer.

Eine besonders gute Resorption des Implantats wird sichergestellt, wenn das mindestens eine körperverträgliche Material Gelatine ist.

Vorzugsweise kann das mindestens eine körperverträgliche Material auch ein kollagenes Gel sein.

Denkbar ist es ferner, daß das mindestens eine körperverträgliche Material ein Chitinderivat ist.

Vorteilhaft ist es, wenn das mindestens eine Körperverträgliche Material eine Hyaluronsäurederivat ist.

Damit sich das Implantat nicht in unerwünschter Weise vom Defektbereich lösen kann, ist es vorteilhaft, wenn Verbindungsmittel vorgesehen sind zum Verankern des Implantats am defekten Knorpelbereich.

Besonders einfach kann das Implantat am defekten Knorpelbereich verankert werden, wenn die Verbindungsmittel resorbierbares Nahtmaterial umfassen. Das Implantat kann so in den Defektbereich eingenäht werden.

Zusätzlich oder alternativ können die Verbindungsmittel mindestens einen resorbierbaren Befestigungsstift umfassen. Mit dem mindestens einen Befestigungsstift läßt sich das Implantat zumindest temporär am defekten Knorpelbereich festlegen. Zusätzlich oder alternativ können die Verbindungsmittel einen körperverträglichen und resorbierbaren Klebstoff umfassen. Die Verwendung eines Klebstoffs gestattet es, das Implantat in vorteilhafter Weise großflächig am Rand des natürlichen Knorpels festzulegen. Die Resorptionszeit aller möglicher Verbindungsmittel ist vorzugsweise so gewählt, daß die Verbindungsmittel erst dann vollständig resorbiert sind, wenn ein minimales Einwachsen des Implantats in den defekten Knorpelbereich gewährleistet ist.

Um ein Einbluten von körpereigenen Stammzellen in den Zellträger zu erleichtern, kann es günstig sein, wenn die Defektanlagefläche des Zellträgers perforiert ist.

Die eingangs gestellte Aufgabe wird bei einem Verfahren der eingangs beschriebenen Art zum Herstellen eines Knorpelersatzimplantats erfindungsgemäß dadurch gelöst, daß zur Ausbildung des Zellträgers mindestens zwei voneinander unabhängige Zellträgerelemente verwendet werden und daß jedes der mindestens zwei Zellträgerelemente fest mit dem Träger verbunden wird. Denkbar wäre es auch, ein einheitliches Implantatmaterial zu wählen und einen Teil des Materials so zu bearbeiten, daß zwei unterschiedliche Implantatbereiche ausgebildet werden, nämlich ein Zellträger und ein Träger. Sowohl das Verbinden zweier unterschiedlicher Elemente als auch das Bearbeiten eines einheitlichen Elements ist besonders einfach. Das Verbinden von zwei unterschiedlichen Elementen hat zusätzlich den Vorteil, daß auf einfache Weise unterschiedliche Materialien verwendet werden können. Da der Zellträger infolge seiner Resorption kontrahieren kann, das heißt an Volumen einbüßt, wird zur Vermeidung der Ausbildung von großen Kontraktionsspalten im Randbereich des Implantats vorgeschlagen, daß zur Ausbildung des Zellträgers mindestens zwei voneinander unabhängige Zellträgerelemente verwendet werden und daß jedes der mindestens zwei Zellträgerelemente fest mit dem Träger verbunden wird. Auf diese Weise können die einzelnen Zellträgerelemente kontrahieren und schrumpfen, was zwar Kontraktionsspalten zwischen benachbarten Zellträgerelementen zur Folge hat, allerdings sind diese insgesamt jeweils deutlich kleiner als ein sich ohne vorhandene Zellträgerelemente ausbildender Kontraktionsspalt im Randbereich des Implantats.

Um das Einwachsen des Implantats in einen defekten Knorpelbereich zu erleichtern und um Abstoßungsreaktionen des menschlichen Körpers zu vermeiden, ist es vorteilhaft, wenn zum Herstellen des Implantats mindestens ein körperverträgliches Material verwendet wird.

Günstig ist es, wenn der Zellträger mit menschlichen Zellen beimpft wird. Das Impfen des Zellträgers mit Zellen vor der Implantation hat den Vorteil, daß so sichergestellt werden kann, daß ausreichend Zellmaterial in den defekten Knorpelbereich implantiert wird. Beim Einbluten von Stammzellen besteht unter Umständen das Risiko, daß nicht genügend Zellen einbluten und so eine nicht optimale Regeneration des defekten Knorpelbereichs erreicht wird.

Vorzugsweise werden als menschlichen Zellen aus körpereigenen Zellen gezüchtete und vermehrte Chondrocyten verwendet. Derartige Zellen werden vom Körper nicht abgestoßen und gewährleisten einen raschen Aufbau von neuer Knorpelsubstanz.

Damit die Stabilität des Implantats insgesamt erhöht wird, ist es vorteilhaft, wenn der Zellträger unlösbar mit dem Träger verbunden wird.

Vorteilhaft ist es, wenn die mindestens zwei Zellträgerelemente nicht direkt, sondern nur über den Träger miteinander verbunden werden. Auf diese Weise kann jedes Zellträgerelement für sich kontrahieren, was nur zu einer lokalen Spaltbildung um jeweils ein Zellträgerelement herum führt, die Ausbildung eines großen und unerwünschten Kontraktionsspalts wird dadurch vermieden. Zudem werden alle Kontraktionsspalte zwischen einzelnen Zellträgerelementen durch den Träger bedeckt, so daß in jedem Fall eine durchgehende Gelenkfläche gewährleistet ist.

Besonders einfach läßt sich ein Knorpelersatzimplantat herstellen, wenn die mindestens zwei Zellträgerelemente durch mindestens einen Einschnitt in einen einstückigen Zellträger hergestellt werden, so daß die mindestens zwei Trägerelemente durch mindestens einen Schnittspalt getrennt sind. Beispielsweise können die Zellträger mit einem scharfen Schneidwerkzeug, z.B. Skalpell oder dergleichen, eingeschnitten werden, so daß ein minimal breiter Schnittspalt ausgebildet wird.

Gemäß einer bevorzugten Variante des Verfahrens kann vorgesehen sein, daß die mindestens zwei Zellträgerelemente durch mindestens eine Spalte getrennt werden, welche mindestens eine Breite in einem Bereich von 0,1 bis 0,8 mm aufweist. Die Ausbildung einer solchen Spalte ermöglicht es, da Implantat auch in einen gekrümmten Bereich einzusetzen, wobei dann benachbarte Zellträgerelemente wiederum aneinander anliegen können.

Um den defekten Knorpelbereich optimal und vollständig ausfüllen zu können, ist es von Vorteil, wenn die mindestens eine Spalte mit einem körperverträglichen, resorbierbaren Material aufgefüllt wird.

Damit die Ausbildung von Verwerfungen durch auf den Zellträger in Folge seiner Resorption wirkenden Zugkräfte vermieden werden kann, ist es vorteilhaft, wenn der Zellträger zur Herstellung des mindestens einen Schnittspalts oder der mindestens einen Spalte mindestens bis zur Hälfte der Schichtdicke des Zellträgers eingeschnitten wird. Dadurch wird sichergestellt, daß einzelne Zellträgerelemente lokal kontrahieren können, so daß zwar um jedes einzelne Zellträgerelement herum Kontraktionsspalte entstehen, das Implantat insgesamt jedoch an seiner Position verbleibt und die Ausbildung eines breiten Kontraktionsspalts im Randbereich des Implantats vermieden wird.

Die eingangs gestellte Aufgabe wird ferner gelöst durch ein nicht erfindungsgemäßes Verfahren zur Behandlung eines traumatischen oder entzündlich degenerierten Knorpeldefekts an einem Knorpel eines menschlichen Körpers, und zwar dadurch, daß in den Knorpeldefekt eines der oben beschriebenen Knorpelersatzimplantate eingesetzt wird. Dadurch wird sichergestellt, daß sich im Randbereich des Implants nach Implantation und Resorption desselben kein großer Kontraktionsspalt ausbildet, welcher als mögliche biomechanische Schwachstelle den Ausgangspunkt weiterer Knorpeldegeneration darstellen kann.

Um sicherzustellen, daß das Implantat seine Position am defekten Knorpelbereich darstellt, ist es von Vorteil, wenn das Knorpelersatzimplantat mit resorbierbarem Nahtmaterial an dem Knorpeldefekt verankert wird.

Alternativ oder zusätzlich kann das Knorpelersatzimplantat durch Festlegen mit mindestens einem resorbierbaren Befestigungspin an dem Knorpeldefekt verankert werden. Derartige Befestigungspins erleichtern das Einnähen des Implantats am defekten Knorpelbereich.

Um einen optimalen Halt des Implantats am defekten Knorpelbereich sicherzustellen, ist es günstig, wenn das Knorpelersatzimplantat durch Einkleben mit einem körperverträglichen und resorbierbaren Klebstoff an dem Knorpeldefekt verankert wird. So kann sichergestellt werden, daß der Zellträger einerseits am Träger und andererseits am defekten Knorpelbereich festgelegt ist.

Das Verfahren eignet sich zur Wiederherstellung von defekten Knorpelbereichen im Knie, in der Bandscheibe oder in jedem anderen Gelenk des menschlichen Körpers.

Grundsätzlich ist es von Vorteil, wenn das Implantat vor der Implantation mit Knorpelzellen, beispielsweise aus körpereigenen Zellen, gezüchteten und vermehrten Chondrocyten beimpft wird. Günstig ist es jedoch, wenn das Knorpelersatzimplantat ohne vorherige Besiedelung des Zellträgers oder des Trägers implantiert wird, um ein Einbluten von Stammzellen aus dem Knochenmark in das Knorpelersatzimplantat zu ermöglichen. Eingeblutete Stammzellen können sich dann zum Beispiel in Knorpelzellen umwandeln und zur Regeneration des defekten Knorpelbereichs dienen.

Vorzugsweise wird vor dem Einsetzen des Knorpelersatzimplantats eine subchondrale Platte am Knochen perforiert. Insbesondere dann, wenn ein Knorpelersatzimplantat verwendet wird, welches einen Zellträger aufweist, in welchen vor Implantation des Implantats keine Zellen implantiert werden, bewirkt eine Perforation der subchondralen Platte am Knochen, also der Knochenoberfläche, an welcher eine Defektanlagefläche des Zellträgers nach Implantation anliegt, daß körpereigene Stammzellen durch die Perforation bedingtes Bluten in den Zellträger einbluten können.

Die nachfolgende Beschreibung bevorzugter Ausführungsformen der Erfindung dient im Zusammenhang mit der Zeichnung der näheren Erläuterung. Es zeigen:
- Figur 1:: einen Querschnitt durch ein eingewachsene Knorpelersatzimplantat, wie es aus dem Stand der Technik bekannt ist;
- Figur 2:: eine perspektivische Ansicht eines ersten Ausführungsbeispiels eines erfindungsgemäßen Implantats vor dem Einsetzen in einen defekten Knorpelbereich;
- Figur 3:: eine Längsschnittansicht durch das in den defekten Knorpelbereich eingesetzte, in Figur 2 dargestellte Implantat;
- Figur 4:: eine Schnittansicht ähnlich Figur 3 nach einsetzender Resorption der Zellträgerschicht; und
- Figur 5:: eine Schnittansicht ähnlich Figur 3 durch ein zweites Ausführungsbeispiel eines jedoch nicht erfindungsgemäßen Implantats mit Pins als Verankerungselemente zum Verankern des Implantats im Defekt.

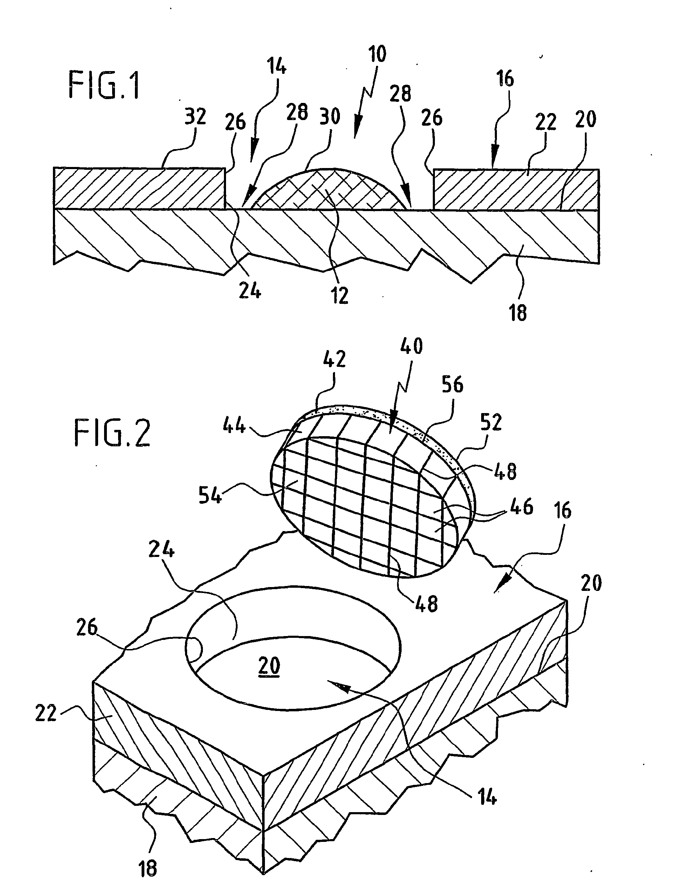

In Figur 1 ist ein Knorpelersatzimplantat 10 dargestellt, wie es aus dem Stand der Technik bekannt ist. Es besteht lediglich aus einem Zellträger 12, der vor Implantation mit Zellen beimpft werden kann oder auch nicht. In Figur 1 ist das Knorpelersatzimplantat 10 in einen Knorpeldefekt 14 eines ansonsten intakten Knorpelbereichs 16 eines Gelenks eines menschlichen Körpers eingesetzt. Der Knorpelbereich 16 wird insgesamt gebildet durch einen einen Knochen 18 und dessen Oberfläche 20 bedeckenden Gelenkknorpel 22. Der Aufbau des Knochens 18 und dessen Oberfläche 20 bedeckenden Gelenkknorpels 22 ist der Einfachheit halber als Zweischichtmodell dargestellt. In der Natur erfolgt der Übergang von Knochen zu Knorpel üblicherweise über einen Gradienten von mehreren Millimetern Länge. Der Knorpeldefekt 14 kann beispielsweise aufgrund traumatischer oder entzündlich degenerativer Prozesse entstanden sein. Wie in der Figur 1 schematisch dargestellt, bildet der Knorpeldefekt 14 eine Lücke im Knorpelbereich 16, welche seitlich durch Knorpelränder 26 des intakten Knorpelbereichs 16 begrenzt wird. Die Knorpelränder 26 sind im wesentlichen glatt und entstehen durch Entfernen degenerativen Knorpels vor dem Einsetzen des Knorpelersatzimplantats 10.

In Figur 1 ist das Ersatzimplantat nach Einwachsen, das heißt nach mindestens teilweiser Resorption des Implantats dargestellt. Der Zellträger bildet infolge der Kontraktion eine vom Knochen 18 weg weisende gewölbte Zellträgerfläche 30. Der Zellträger 12 zieht sich insgesamt zusammen, so daß ein den Zellträger 12 umgebender Kontraktionsspalt 28 ausgebildet wird, der sich zwischen den Knorpelrändern 26 und der in Richtung auf diese weisenden Zellträgerfläche 30 des Zellträgers 12 ausbildet. Eine solche persistierende Spaltbildung im Übergangsbereich zwischen intaktem Knorpelbereich 16 und Knorpelersatzimplantat 10 bildet als biomechanische Schwachstelle häufig den Ausgangspunkt weiterer Knorpeldegenerationen.

In den nachfolgenden Figuren werden der Einfachheit halber identische Bezugszeichen zur Beschreibung des Knorpeldefekts verwendet.

In Figur 2 ist ein erfindungsgemäßes Knorpelersatzimplantat dargestellt und insgesamt mit dem Bezugszeichen 40 versehen. Es ist vor der Implantation im wesentlichen scheibenförmig ausgebildet und weist eine äußere Kontur auf, welche an den Knorpeldefekt 14 angepaßt ist, um die Lücke 24 im Knorpelbereich 16 möglichst vollständig auszufüllen.

Das Knorpelersatzimplantat 40 ist insgesamt zweiphasig aufgebaut, das heißt, es umfaßt eine Trägerschicht 42 und eine flächig mit dieser verbundene Zellträgerschicht 44. Die Trägerschicht 42 ist dünner als die Zellträgerschicht 44 und weist typischerweise eine Dicke von 0,01 mm bis 0,8 mm auf, die Zellträgerschicht eine Dicke im Bereich von 0,3 bis 3,5 mm. Schichtdicken der Trägerschicht 42 und der Zellträgerschicht 44 werden typischerweise in Abhängigkeit der Tiefe des Knorpeldefekts 14 gewählt.

Sowohl die Trägerschicht 42 als auch die Zellträgerschicht 44 sind jeweils aus einem körperverträglichen resorbierbaren Material gefertigt. Die mechanische Stabilität der Trägerschicht 42 ist größer als die der Zellträgerschicht 44, ebenso ist die Resorptionszeit der Trägerschicht 42 größer als die der Zellträgerschicht 44. Die Trägerschicht 42 weist zudem eine dichtere Struktur auf als die Zellträgerschicht 44. Letztere ist schwammartig ausgebildet und weist eine Porenweite von 30 bis 500 µm auf. Die Trägerschicht 42 dagegen weist Porenweiten von maximal 20 µm auf.

Die makroskopische Struktur der Zellträgerschicht 44 wird definiert durch eine Vielzahl von quader- oder würfelförmigen Zellträgerelementen in Form von Parzellen 46, welche gebildet werden durch Einschneiden der Zellträgerschicht in Richtung auf die Trägerschicht 42 hin. Die Parzellen 46 werden somit durch Schnittspalte 48 voneinander getrennt und sind nur über die Trägerschicht 42 miteinander verbunden. Beispielsweise können die einzelnen Parzellen 46 durch Kleben mit der Trägerschicht 42 verbunden sein.

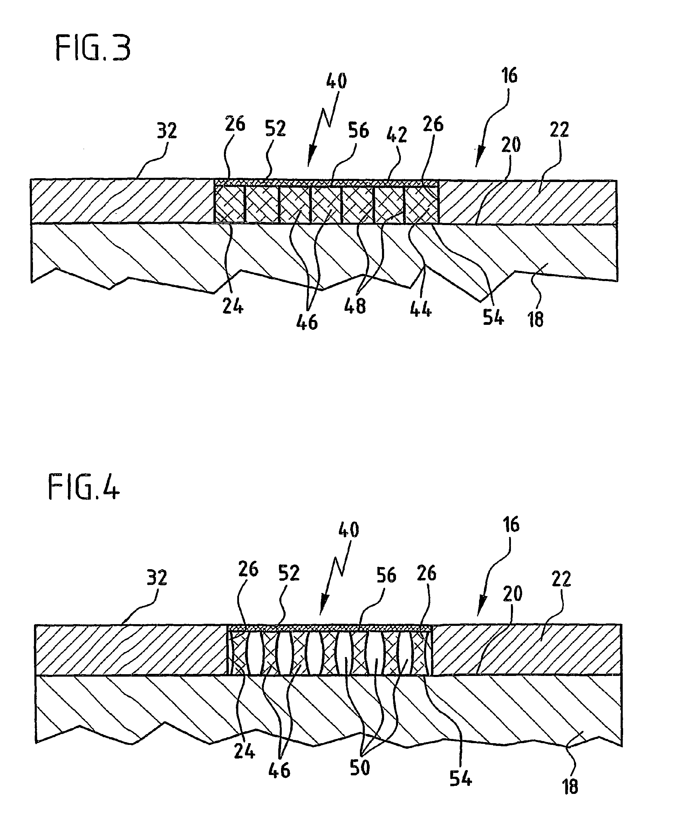

In Figur 3 ist das in den Knorpeldefekt 14 eingesetzte Knorpelersatzimplantat 40 im Längsschnitt dargestellt. Es füllt die Lücke 24 praktisch vollständig aus. Eine von der Trägerschicht 42 weg weisende Knochenanlagefläche 54 der Zellträgerschicht 44 liegt im wesentlichen direkt an der Oberfläche 20 des Knochens 18 an.

Nach Einsetzen der Resorption der Zellträgerschicht 44 kontrahieren die einzelnen Parzellen 46, wodurch sich die Schnittspalte 48 zu Kontraktionsspalten 50 vergrößern. Durch die feste Verbindung der Zellträgerschicht 44 mit der Trägerschicht 42 und durch Anwachsen der Parzellen 46 an die Oberfläche 20 des Knochens 20 nehmen die Parzellen 46 eine kühlturmartige Form an. Durch die größere mechanische Stabilität der Trägerschicht 42 behält diese jedoch ihre ursprüngliche Form wesentlich länger bei als die Zellträgerschicht 44, so daß die Zellträgeroberfläche 52, welche im Bereich des Knorpeldefekts 14 die Knorpeloberfläche 32 des Knorpelbereichs 16 vervollständigt, unverändert in ihrer implantierten Position bleibt. Die Knorpeloberfläche 32, welche eine Gelenkoberfläche des Knochens 18 bildet, bleibt somit auch nach Einsetzen der Resorption der Zellträgerschicht 44 intakt, anders als dies bei aus dem Stand der Technik bekannten Implantaten der Fall und in Figur 1 dargestellt ist. Kontraktionsspalte zwischen Parzellen 46 im Randbereich des Knorpelersatzimplantats 40 und aufgrund von entsprechender Bearbeitung glatter Knorpelrändern 26 des intakten Knorpelbereichs 16 entstehen zwar auch, sind jedoch so klein, daß sie keinen Angriffspunkt und somit keine biomechanische Schwachstelle ausbilden.

War die Zellträgerschicht 44 vor Implantation mit Zellen, beispielsweise aus körpereigenen Zellen gezüchteten und vermehrten Chondrocyten beimpft, so können diese eine neue Knorpelschicht aufbauen, die nach vollständiger Resorption der Zellträgerschicht 44 und der Trägerschicht 42 die Lücke vollständig ausfüllt.

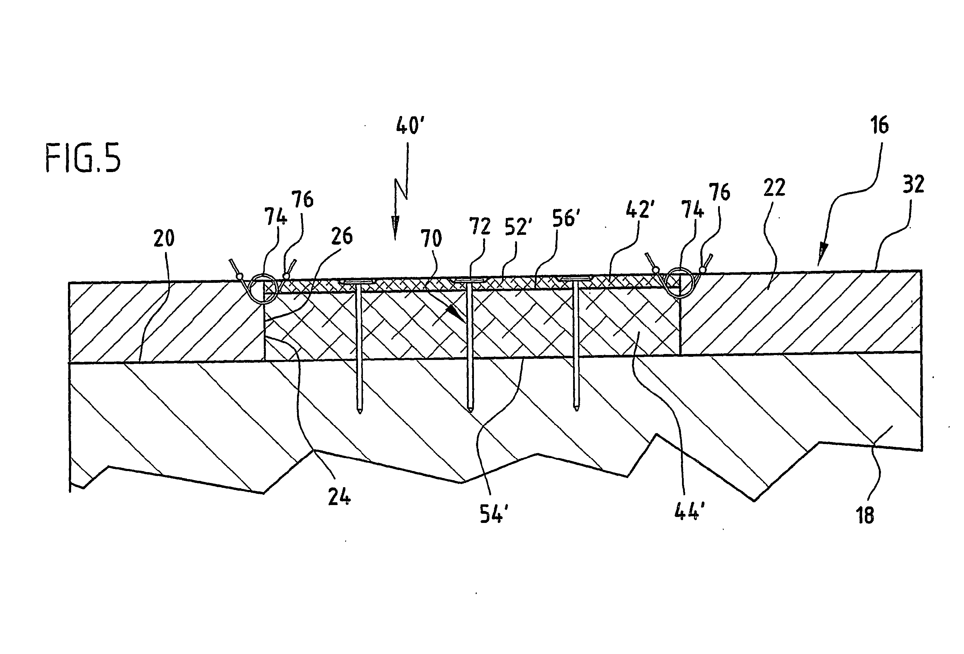

In Figur 5 ist ein zweites, insgesamt mit dem Bezugszeichen 40' versehenes, jedoch nicht erfindungsgemäßes Knorpelersatzimplantat dargestellt. Es ähnelt in seinem grundsätzlichen Aufbau dem Knorpelersatzimplantat 40, so daß identische, mit einem " ' " versehene Bezugszeichen verwendet werden. Es umfaßt eine Trägerschicht 42', welche flächig an einer Zellträgerschicht 44' anliegt und mit dieser verbunden ist. Anders als die Zellträgerschicht 44 ist die Zellträgerschicht 44' nicht in einzelne Parzellen 46 unterteilt, sondern verbleibt einstückig. Denkbar wäre es jedoch auch, die Zellträgerschicht 44' mit Schnittspalten 48 zu versehen, so daß einzelne Parzellen 46 ausgebildet werden.

Zur Fixierung des Knorpelersatzimplantats 40' wird eine Knochenanlagefläche 54' der Zellträgerschicht 44' mit der Oberfläche 20 des Knochens 20 verklebt. Alternativ wird nur eine am gesunden Knorpelrand 26 anliegende Ringfläche des Trägers 42 mit dem Knorpelrand 26 verklebt. Alternativ oder zusätzlich können Haltepins 70 vorgesehen sein, die einen an der Trägeroberfläche 52' anliegenden Kopf 72 aufweisen, das gesamte Knorpelersatzimplantat 40' durchsetzen und im Knochen 18 verankert werden.

Alternativ oder zusätzlich kann das Knorpelersatzimplantat 40' im Bereich der Knorpelränder 26 mit dem intakten Knorpelbereich 16 vernäht werden. Hierzu werden resorbierbare Fäden 74 eingesetzt und das Knorpelersatzimplantat 40' durch entsprechende Ausbildung von Knoten 76 an der Knorpeloberfläche 32 und der Trägeroberfläche 52' fixiert. Sowohl die verwendeten Haltepins 70 als auch der verwendete Klebstoff sind vorzugsweise aus einem körperverträglichen resorbierbaren Material.

Die Haltepins 70 sowie das beschriebene Nahtmaterial und der Klebstoff können auch in Verbindung mit dem Knorpelersatzimplantat 40 verwendet werden.

## Patentansprüche

1. Knorpelersatzimplantat (40) zur biologischen Regeneration eines geschädigten Knorpelbereichs (14) eines Gelenkknorpels (16) eines menschlichen Körpers, umfassend einen eine Defektanlagefläche (54) zur Anlage an den geschädigten Knorpelbereich (14) aufweisenden Zellträger (44), welcher derart ausgebildet und aufgebaut ist, daß er mit menschlichen Zellen besiedelt werden kann, wobei der Zellträger (44) mit einer von der Defektanlagefläche (54) weg weisenden Zellträgerfläche flächig an einem Träger (42) anliegt und mit dem Träger (42) verbunden ist, **dadurch gekennzeichnet, daß** der Zellträger (44) mindestens zwei voneinander unabhängige Zellträgerelemente (46) umfaßt und daß jedes der mindestens zwei Zellträgerelemente (46) mit dem Träger (42) verbunden ist.

2. Implantat nach Anspruch 1, **dadurch gekennzeichnet, daß** das Implantat (40) aus mindestens einem körperverträglichen Material hergestellt ist.

3. Implantat nach einem der voranstehenden Ansprüche, **dadurch gekennzeichnet, daß** das mindestens eine körperverträgliche Material resorbierbar ist.

4. Implantat nach einem der Ansprüche 2 oder 3, **dadurch gekennzeichnet, daß** der Zellträger (44) und der Träger (42) aus unterschiedlichen körperverträglichen Materialien hergestellt sind.

5. Implantat nach einem der voranstehenden Ansprüche, **dadurch gekennzeichnet, daß** der Zellträger (44) und der Träger (42) unterschiedlich lange Resorptionszeiten aufweisen.

6. Implantat nach Anspruch 5, **dadurch gekennzeichnet, daß** die Resorptionszeit des Trägers (42) länger ist als die Resorptionszeit des Zellträgers (44).

7. Implantat nach einem der voranstehenden Ansprüche, **dadurch gekennzeichnet, daß** der Zellträger (44) und der Träger (42) unterschiedliche Resorptionskinetiken aufweisen.

8. Implantat nach einem der voranstehenden Ansprüche, **dadurch gekennzeichnet, daß** der Träger (42) derart ausgebildet und aufgebaut ist, daß er mit menschlichen Zellen besiedelt werden kann.

9. Implantat nach einem der voranstehenden Ansprüche, **dadurch gekennzeichnet, daß** der Zellträger (44) mindestens zwei unterschiedliche Zellträgerschichten umfaßt.

10. Implantat nach einem der voranstehenden Ansprüche, **dadurch gekennzeichnet, daß** der Zellträger (44) mit menschlichen Zellen beimpft ist.

11. Implantat nach Anspruch 10, **dadurch gekennzeichnet, daß** die menschlichen Zellen aus körpereigenen Zellen gezüchtete und vermehrte Chondrocyten sind.

12. Implantat nach einem der voranstehenden Ansprüche, **dadurch gekennzeichnet, daß** der Zellträger (44) und der Träger (42) eine unterschiedliche mechanische Stabilität aufweisen.

13. Implantat nach Anspruch 12, **dadurch gekennzeichnet, daß** der Träger (42) eine höhere mechanische Stabilität als der Zellträger (44) aufweist.

14. Implantat nach einem der voranstehenden Ansprüche, **dadurch gekennzeichnet, daß** der Zellträger (44) und der Träger (42) unterschiedliche Elastizitätsmodule aufweisen.

15. Implantat nach Anspruch 14, **dadurch gekennzeichnet, daß** der Elastizitätsmodul des Trägers (42) größer ist als der Elastizitätsmodul des Zellträgers (44).

16. Implantat nach einem der voranstehenden Ansprüche, **dadurch gekennzeichnet, daß** der Träger (42) eine dichtere Struktur als der Zellträger (44) aufweist.

17. Implantat nach einem der voranstehenden Ansprüche, **dadurch gekennzeichnet, daß** der Zellträger (44) unlösbar mit dem Träger (42) verbunden ist.

18. Implantat nach einem der voranstehenden Ansprüche, **dadurch gekennzeichnet, daß** der Zellträger (44) eine schwammartige Struktur aufweist.

19. Implantat nach einem der voranstehenden Ansprüche, **dadurch gekennzeichnet, daß** der Zellträger (44) eine Zellträgerschichtdicke in einem Bereich von 0,3 bis 3,5 mm aufweist.

20. Implantat nach Anspruch 19, **dadurch gekennzeichnet, daß** der Zellträger (44) eine Zellträgerschichtdicke in einem Bereich von 1,0 mm bis 3,5 mm aufweist.

21. Implantat nach einem der voranstehenden Ansprüche, **dadurch gekennzeichnet, daß** der Träger (42) eine Trägerschichtdicke in einem Bereich von 0,01 mm bis 0,8 mm aufweist.

22. Implantat nach Anspruch 21, **dadurch gekennzeichnet, daß** der Träger (42) eine Trägerschichtdicke in einem Bereich von 0,2 mm bis 0,4 mm aufweist.

23. Implantat nach einem der voranstehenden Ansprüche, **dadurch gekennzeichnet, daß** der Zellträger (44) aus einem Material hergestellt ist, welches eine Porenweite in einem Bereich von 30 µm bis 500 µm aufweist.

24. Implantat nach Anspruch 23, **dadurch gekennzeichnet, daß** der Zellträger (44) aus einem Material hergestellt ist, welches eine Porenweite im Bereich von 80 µm bis 150 µm aufweist.

25. Implantat nach einem der voranstehenden Ansprüche, **dadurch gekennzeichnet, daß** der Träger (42) aus einem porösen Material hergestellt ist, welches eine Porenweite von maximal 20 µm aufweist.

26. Implantat nach einem der voranstehenden Ansprüche, **dadurch gekennzeichnet, daß** die mindestens zwei Zellträgerelemente (46) Zellträgerelementflächen aufweisen, und daß Zellträgerelementflächen benachbarter Zellträgerelemente (46) lose aneinander angrenzen.

27. Implantat nach einem der voranstehenden Ansprüche, **dadurch gekennzeichnet, daß** die mindestens zwei Zellträgerelemente (46) durch mindestens einen Schnittspalt (48) getrennt sind, welcher durch Einschneiden eines einstückigen Zellträgers (44) hergestellt ist.

28. Implantat nach einem der Ansprüche 1 bis 26, **dadurch gekennzeichnet, daß** die mindestens zwei Zellträgerelemente (46) durch mindestens eine Spalte (48) getrennt sind und daß die mindestens eine Spalte (48) eine Breite in einem Bereich von 0,1 bis 0,8 mm aufweist.

29. Implantat nach Anspruch 28, **dadurch gekennzeichnet, daß** die mindestens eine Spalte (48) mit einem körperverträglichen, resorbierbaren Material aufgefüllt ist.

30. Implantat nach einem der voranstehenden Ansprüche, **dadurch gekennzeichnet, daß** der mindestens eine Schnittspalt (48) oder die mindestens eine Spalte eine Tiefe aufweisen, welche mindestens der halben Zellträgerschichtdicke des Zellträgers (44) entspricht.

31. Implantat nach einem der Ansprüche 2 bis 30, **dadurch gekennzeichnet, daß** das mindestens eine körperverträgliche Material ein kollagenes Vlies ist.

32. Implantat nach einem der Ansprüche 2 bis 31, **dadurch gekennzeichnet, daß** das mindestens eine körperverträgliche Material ein synthetisches Polymer ist.

33. Implantat nach einem der Ansprüche 2 bis 32, **dadurch gekennzeichnet, daß** das mindestens eine körperverträgliche Material Gelatine ist.

34. Implantat nach einem der Ansprüche 2 bis 33, **dadurch gekennzeichnet, daß** das mindestens eine körperverträgliche Material ein kollagenes Gel ist.

35. Implantat nach einem der Ansprüche 2 bis 34, **dadurch gekennzeichnet, daß** das mindestens eine körperverträgliche Material ein Chitinderivat ist.

36. Implantat nach einem der Ansprüche 2 bis 35, **dadurch gekennzeichnet, daß** das mindestens eine körperverträgliche Material ein Hyaluronsäurederivat ist.

37. Implantat nach einem der voranstehenden Ansprüche, **dadurch gekennzeichnet, daß** Verbindungsmittel (70, 74) vorgesehen sind zum Verankern des Implantats (40) am defekten Knorpelbereich (14).

38. Implantat nach Anspruch 37, **dadurch gekennzeichnet, daß** die Verbindungsmittel resorbierbares Nahtmaterial (74) umfassen.

39. Implantat nach einem der Ansprüche 37 oder 38, **dadurch gekennzeichnet, daß** die Verbindungsmittel mindestens einen resorbierbaren Befestigungsstift (70) umfassen.

40. Implantat nach einem der Ansprüche 37 bis 39, **dadurch gekennzeichnet, daß** die Verbindungsmittel einen körperverträglichen und resorbierbaren Klebstoff umfassen.

41. Implantat nach einem der voranstehenden Ansprüche, **dadurch gekennzeichnet, daß** die Defektanlagefläche (54) des Zellträgers (44) perforiert ist.

42. Verfahren zum Herstellen eines Knorpelersatzimplantats zur biologischen Regeneration eines geschädigten Gelenkknorpels eines menschlichen Körpers, wobei ein eine Defektanlagefläche zur Anlage an den geschädigten Knorpelbereich aufweisender Zellträger verwendet wird, welcher derart ausgebildet und aufgebaut ist, daß er mit menschlichen Zellen besiedelt werden kann, wobei der Zellträger mit einer von der Defektanlagefläche weg weisenden Zellträgerfläche flächig mit einem Träger verbunden wird, **dadurch gekennzeichnet, daß** zur Ausbildung des Zellträgers mindestens zwei voneinander unabhängige Zellträgerelemente verwendet werden und daß jedes der mindestens zwei Zellträgerelemente fest mit dem Träger verbunden wird.

43. Verfahren nach Anspruch 42, **dadurch gekennzeichnet, daß** zum Herstellen des Implantats mindestens ein körperverträgliches Material verwendet wird.

44. Verfahren nach einem der Ansprüche 42 oder 43, **dadurch gekennzeichnet, daß** der Zellträger mit menschlichen Zellen beimpft wird.

45. Verfahren nach Anspruch 44, **dadurch gekennzeichnet, daß** als menschliche Zellen aus körpereigenen Zellen gezüchtete und vermehrte Chondrocyten verwendet werden.

46. Verfahren nach einem der Ansprüche 42 bis 45, **dadurch gekennzeichnet, daß** der Zellträger unlösbar mit dem Träger verbunden wird.

47. Verfahren nach einem der Ansprüche 42 bis 46, **dadurch gekennzeichnet, daß** die mindestens zwei Zellträgerelemente nicht direkt, sondern nur über den Träger miteinander verbunden werden.

48. Verfahren nach einem der Ansprüche 42 bis 47, **dadurch gekennzeichnet, daß** die mindestens zwei Zellträgerelemente durch mindestens einen Einschnitt in einen einstückigen Zellträger hergestellt werden, so daß die mindestens zwei Trägerelemente durch mindestens einen Schnittspalt getrennt sind.

49. Verfahren nach einem der Ansprüche 42 bis 48, **dadurch gekennzeichnet, daß** die mindestens zwei Zellträgerelemente durch mindestens eine Spalte getrennt werden, welche mindestens eine Breite in einem Bereich von 0,1 bis 0,8 mm aufweist.

50. Verfahren nach Anspruch 49, **dadurch gekennzeichnet, daß** die mindestens eine Spalte mit einem körperverträglichen, resorbierbaren Material aufgefüllt wird.

51. Verfahren nach einem der Ansprüche 48 bis 50, **dadurch gekennzeichnet, daß** der Zellträger zur Herstellung des mindestens einen Schnittspalts oder der mindestens einen Spalte mindestens bis zur Hälfte der Schichtdicke des Zellträgers eingeschnitten wird.

## Claims

1. Cartilage replacement implant (40) for the biological regeneration of a damaged cartilage area (14) of articular cartilage (16) in the human body, comprising a cell carrier (44) which has a defect-contacting surface (54) for placement on the damaged cartilage area (14) and is formed and designed for colonization with human cells, the cell carrier (44) resting with surface-to-surface contact on a carrier (42) at a cell carrier surface that faces away from the defect-contacting surface (54), and being joined to the carrier (42), **characterized in that** the cell carrier (44) comprises at least two cell carrier elements (46) which are independent of each other, and **in that** each of the at least two cell carrier elements (46) is joined to the carrier (42).

2. Implant in accordance with claim 1, **characterized in that** the implant (40) is made from at least one biocompatible material.

3. Implant in accordance with any one of the preceding claims, **characterized in that** the at least one biocompatible material is resorbable.

4. Implant in accordance with claim 2 or 3, **characterized in that** the cell carrier (44) and the carrier (42) are made from different biocompatible materials.

5. Implant in accordance with any one of the preceding claims, **characterized in that** the cell carrier (44) and the carrier (42) have resorption times of different lengths.

6. Implant in accordance with claim 5, **characterized in that** the resorption time of the carrier (42) is longer than the resorption time of the cell carrier (44).

7. Implant in accordance with any one of the preceding claims, **characterized in that** the cell carrier (44) and the carrier (42) have different resorption kinetics.

8. Implant in accordance with any one of the preceding claims, **characterized in that** the carrier (42) is formed and designed for colonization with human cells.

9. Implant in accordance with any one of the preceding claims, **characterized in that** the cell carrier (44) comprises at least two different cell carrier layers.

10. Implant in accordance with any one of the preceding claims, **characterized in that** the cell carrier (44) is inoculated with human cells.

11. Implant in accordance with claim 10, **characterized in that** the human cells are chondrocytes that are cultured and proliferated from the body's own cells.

12. Implant in accordance with any one of the preceding claims, **characterized in that** the cell carrier (44) and the carrier (42) have a different mechanical stability.

13. Implant in accordance with claim 12, **characterized in that** the carrier (42) has a higher mechanical stability than the cell carrier (44).

14. Implant in accordance with any one of the preceding claims, **characterized in that** the cell carrier (44) and the carrier (42) have different moduli of elasticity.

15. Implant in accordance with claim 14, **characterized in that** the modulus of elasticity of the carrier (42) is greater than the modulus of elasticity of the cell carrier (44).

16. Implant in accordance with any one of the preceding claims, **characterized in that** the carrier (42) has a denser structure than the cell carrier (44).

17. Implant in accordance with any one of the preceding claims, **characterized in that** the cell carrier (44) is undetachably connected to the carrier (42).

18. Implant in accordance with any one of the preceding claims, **characterized in that** the cell carrier (44) has a sponge-like structure.

19. Implant in accordance with any one of the preceding claims, **characterized in that** the cell carrier (44) has a cell carrier layer thickness ranging from 0.3 mm to 3.5 mm.

20. Implant in accordance with claim 19, **characterized in that** the cell carrier (44) has a cell carrier layer thickness ranging from 1.0 mm to 3.5 mm.

21. Implant in accordance with any one of the preceding claims, **characterized in that** the carrier (42) has a carrier layer thickness ranging from 0.01 mm to 0.8 mm.

22. Implant in accordance with claim 21, **characterized in that** the carrier (42) has a carrier layer thickness ranging from 0.2 mm to 0.4 mm.

23. Implant in accordance with any one of the preceding claims, **characterized in that** the cell carrier (44) is made from a material having a pore width ranging from 30 µm to 500 µm.

24. Implant in accordance with claim 23, **characterized in that** the cell carrier (44) is made from a material having a pore width ranging from 80 µm to 150 µm.

25. Implant in accordance with any one of the preceding claims, **characterized in that** the carrier (42) is made from a porous material having a pore width of at most 20 µm.

26. Implant in accordance with any one of the preceding claims, **characterized in that** the at least two cell carrier elements (46) have cell carrier element surfaces, and **in that** cell carrier element surfaces of adjacent cell carrier elements (46) border loosely on one another.

27. Implant in accordance with any one of the preceding claims, **characterized in that** the at least two cell carrier elements (46) are separated by at least one cut gap (48) made by cutting into a one-piece cell carrier (44).

28. Implant in accordance with any one of claims 1 to 26, **characterized in that** the at least two cell carrier elements (46) are separated by at least one split (48), and **in that** the at least one split (48) has a width ranging from 0.1 mm to 0.8 mm.

29. Implant in accordance with claim 28, **characterized in that** the at least one split (48) is filled up with a biocompatible, resorbable material.

30. Implant in accordance with any one of the preceding claims, **characterized in that** the at least one cut gap (48) or the at least one split has a depth corresponding to at least half of the cell carrier layer thickness of the cell carrier (44).

31. Implant in accordance with any one of claims 2 to 30, **characterized in that** the at least one biocompatible material is a collagen non-woven material.

32. Implant in accordance with any one of claims 2 to 31, **characterized in that** the at least one biocompatible material is a synthetic polymer.

33. Implant in accordance with any one of claims 2 to 32, **characterized in that** the at least one biocompatible material is gelatine.

34. Implant in accordance with any one of claims 2 to 33, **characterized in that** the at least one biocompatible material is a collagen gel.

35. Implant in accordance with any one of claims 2 to 34, **characterized in that** the at least one biocompatible material is a chitin derivative.

36. Implant in accordance with any one of claims 2 to 35, **characterized in that** the at least one biocompatible material is a hyaluronic acid derivative.

37. Implant in accordance with any one of the preceding claims, **characterized in that** connection means (70, 74) are provided for anchoring the implant (40) to the defective cartilage area (14).

38. Implant in accordance with claim 37, **characterized in that** the connection means comprise resorbable suture material (74).

39. Implant in accordance with claim 37 or 38, **characterized in that** the connection means comprise at least one resorbable attachment pin (70).

40. Implant in accordance with any one of claims 37 to 39, **characterized in that** the connection means comprise a biocompatible and resorbable adhesive.

41. Implant in accordance with any one of the preceding claims, **characterized in that** the defect-contacting surface (54) of the cell carrier (44) is perforated.

42. Method for producing a cartilage replacement implant for the biological regeneration of damaged articular cartilage in the human body, wherein a cell carrier is used, which has a defect-contacting surface for placement on the damaged cartilage area and which is formed and designed for colonization with human cells, and the cell carrier is joined with surface-to-surface contact to a carrier at a cell carrier surface that faces away from the defect-contacting surface, **characterized in that** at least two cell carrier elements that are independent of each other are used for formation of the cell carrier, and **in that** each of the at least two cell carrier elements is firmly joined to the carrier.

43. Method in accordance with claim 42, **characterized in that** at least one biocompatible material is used for producing the implant.

44. Method in accordance with claim 42 or 43, **characterized in that** the cell carrier is inoculated with human cells.

45. Method in accordance with claim 44, **characterized in that** chondrocytes cultured and proliferated from the body's own cells are used as human cells.

46. Method in accordance with any one of claims 42 to 45, **characterized in that** the cell carrier is undetachably connected to the carrier.

47. Method in accordance with any one of claims 42 to 46, **characterized in that** the at least two cell carrier elements are not directly connected to each other but only via the carrier.

48. Method in accordance with any one of claims 42 to 47, **characterized in that** the at least two cell carrier elements are made by at least one cut into a one-piece cell carrier, so that the at least two carrier elements are separated by at least one cut gap.

49. Method in accordance with any one of claims 42 to 48, **characterized in that** the at least two cell carrier elements are separated by at least one split having at least a width ranging from 0.1 mm to 0.8 mm.

50. Method in accordance with claim 49, **characterized in that** the at least one split is filled up with a biocompatible, resorbable material.

51. Method in accordance with any one of claims 48 to 50, **characterized in that** in order to make the at least one cut gap or the at least one split, the cell carrier is cut into at least as far as half of the layer thickness of the cell carrier.

## Revendications

1. Prothèse de cartilage (40) destinée à la régénération biologique d'une région cartilagineuse endommagée (14) d'un cartilage articulaire (16) d'un corps humain, comprenant un support de cellules (44) présentant une surface d'appui au déficit (54) pour appui contre la zone cartilagineuse endommagée (14), lequel est conçu et construit de telle sorte qu'il peut être ensemencé par des cellules humaines, le support de cellule (44) étant appuyé par une surface de support de cellules opposée à la surface d'appui au déficit (54) en étalement contre un support (42) et étant relié au support (42), **caractérisée en ce que** le support de cellules (44) comprend au moins deux éléments de support de cellules indépendants l'un de l'autre (46) et que chacun des au moins deux éléments de support de cellules (46) est relié au support (42).

2. Prothèse selon la revendication 1, **caractérisée en ce que** la prothèse (40) est en au moins un matériau biocompatible.

3. Prothèse selon l'une des revendications précédentes, **caractérisée en ce que** le au moins un matériau biocompatible est résorbable.

4. Prothèse selon l'une des revendications 2 ou 3, **caractérisée en ce que** le support de cellules (44) et le support (42) sont en des matériaux biocompatibles différents.

5. Prothèse selon l'une des revendications précédentes, **caractérisée en ce que** le support de cellules (44) et le support (42) présentent des durées de résorption de longueurs différentes.

6. Prothèse selon la revendication 5, **caractérisée en ce que** la durée de résorption du support (42) est plus longue que la durée de résorption du support de cellules (44).

7. Prothèse selon l'une des revendications précédentes, **caractérisée en ce que** le support de cellules (44) et le support (42) présentent des cinétiques de résorption différentes.

8. Prothèse selon l'une des revendications précédentes, **caractérisée en ce que** le support (42) est conçu et construit de telle sorte qu'il peut être ensemencé par des cellules humaines.

9. Prothèse selon l'une des revendications précédentes, **caractérisée en ce que** le support de cellules (44) comprend au moins deux couches différentes de support de cellules.

10. Prothèse selon l'une des revendications précédentes, **caractérisée en ce que** le support de cellules (44) est inoculé avec des cellules humaines.

11. Prothèse selon la revendication 10, **caractérisée en ce que** les cellules humaines sont des chondrocytes cultivés et multipliés à partir de cellules endogènes.

12. Prothèse selon l'une des revendications précédentes, **caractérisée en ce que** le support de cellules (44) et le support (42) présentent une stabilité mécanique différente.

13. Prothèse selon la revendication 12, **caractérisée en ce que** le support (42) présente une stabilité mécanique supérieure au support de cellules (44).

14. Prothèse selon l'une des revendications précédentes, **caractérisée en ce que** le support de cellules (44) et le support (42) présentent des modules d'élasticité différents.

15. Prothèse selon la revendication 14, **caractérisée en ce que** le module d'élasticité du support (42) est supérieur au module d'élasticité du support de cellules (44).

16. Prothèse selon l'une des revendications précédentes, **caractérisée en ce que** le support (42) présente une structure plus dense que le support de cellules (44).

17. Prothèse selon l'une des revendications précédentes, **caractérisée en ce que** le support de cellules (44) est relié de façon indétachable au support (42).

18. Prothèse selon l'une des revendications précédentes, **caractérisée en ce que** le support de cellules (44) présente une structure spongieuse.

19. Prothèse selon l'une des revendications précédentes, **caractérisée en ce que** le support de cellules (44) présente une épaisseur de couche de support de cellules de l'ordre de 0,3 à 3,5 mm.

20. Prothèse selon la revendication 19, **caractérisée en ce que** le support de cellules (44) présente une épaisseur de couche de support de cellules de l'ordre de 1,0 mm à 3,5 mm.

21. Prothèse selon l'une des revendications précédentes, **caractérisée en ce que** le support (42) présente une épaisseur de couche de support de cellules de l'ordre de 0,01 mm à 0,8 mm.

22. Prothèse selon la revendication 21, **caractérisée en ce que** le support (42) présente une épaisseur de couche de support de l'ordre de 0,2 mm à 0,4 mm.

23. Prothèse selon l'une des revendications précédentes, **caractérisée en ce que** le support de cellules (44) est fabriqué en un matériau qui présente une taille de pores de l'ordre de 30 µm à 500 µm.

24. Prothèse selon la revendication 23, **caractérisée en ce que** le support de cellules (44) est fabriqué en un matériau qui présente une taille de pores de l'ordre de 80 µm à 150 µm.

25. Prothèse selon l'une des revendications précédentes, **caractérisée en ce que** le support (42) est fabriqué en un matériau poreux qui présente une taille de pores de 20 µm au maximum.

26. Prothèse selon l'une des revendications précédentes, **caractérisée en ce que** les au moins deux éléments de support de cellules (46) présentent des surfaces d'élément de support de cellules et **en ce que** les surfaces d'élément de support de cellules d'éléments de support de cellules (46) voisins sont contiguës de façon lâche.

27. Prothèse selon l'une des revendications précédentes, **caractérisée en ce que** les au moins deux éléments de support de cellules (46) sont séparés par au moins une entaille d'incision (48) qui est produite par incision d'un support de cellules monobloc (44).

28. Prothèse selon l'une des revendications 1 à 26, **caractérisée en ce que** les au moins deux éléments de support de cellules (46) sont séparés par au moins une fente (48) et **en ce que** la au moins une fente (48) présente une largeur de l'ordre de 0,1 à 0,8 mm.

29. Prothèse selon la revendication 28, **caractérisée en ce que** la au moins une fente (48) est empilé d'un matériau résorbable biocompatible.

30. Prothèse selon l'une des revendications précédentes, **caractérisée en ce que** la au moins une entaille d'incision (48) ou la au moins une fente présentent une profondeur qui correspond au moins à la moitié de l'épaisseur de la couche de support de cellules (44).

31. Prothèse selon l'une des revendications 2 à 30, **caractérisée en ce que** le au moins un matériau biocompatible est un non-tissé collagène.

32. Prothèse selon l'une des revendications 2 à 31, **caractérisée en ce que** le au moins un matériau biocompatible est un polymère synthétique.

33. Prothèse selon l'une des revendications 2 à 32, **caractérisée en ce que** le au moins un matériau biocompatible est de la gélatine.

34. Prothèse selon l'une des revendications 2 à 33, **caractérisée en ce que** le au moins un matériau biocompatible est un gel de collagène.

35. Prothèse selon l'une des revendications 2 à 34, **caractérisée en ce que** le au moins un matériau biocompatible est un dérivé de la chitine.

36. Prothèse selon l'une des revendications 2 à 35, **caractérisée en ce que** le au moins un matériau biocompatible est un dérivé de l'acide hyaluronique.

37. Prothèse selon l'une des revendications précédentes, **caractérisée en ce que** sont prévus des moyens de liaison (70, 74) en vue de l'ancrage de la prothèse (40) contre la zone cartilagineuse déficitaire (14).

38. Prothèse selon la revendication 37, **caractérisée en ce que** les moyens de liaison comprennent un matériau de suture résorbable (74).

39. Prothèse selon l'une des revendications 37 ou 38, **caractérisée en ce que** les moyens de liaison comprennent au moins une tige de fixation résorbable (70).

40. Prothèse selon l'une des revendications 37 à 39, **caractérisée en ce que** les moyens de liaison comprennent une colle biocompatible et résorbable.

41. Prothèse selon l'une des revendications précédentes, **caractérisée en ce que** la surface d'appui au déficit (54) du support de cellules (44) est perforée.

42. Procédé de fabrication d'une prothèse de cartilage en vue de la régénération biologique d'un cartilage articulaire endommagé d'un corps humain, un support de cellules présentant une surface d'appui au déficit pour appui contre la zone cartilagineuse endommagée étant utilisé, lequel est conçu et construit de façon qu'il puisse être ensemencé par des cellules humaines, le support de cellules étant relié à un support par une surface de support de cellules opposée à la surface d'appui au déficit en étalement, **caractérisé en ce qu'**on utilise au moins deux éléments de support de cellules indépendants l'un de l'autre pour former le support de cellules et **en ce que** chacun des au moins deux éléments de support de cellules sont reliés solidement au support.

43. Procédé selon la revendication 42, **caractérisé en ce qu'**on utilise pour fabriquer la prothèse au moins un matériau biocompatible.

44. Procédé selon l'une des revendications 42 ou 43, **caractérisé en ce que** le support de cellules est inoculé avec des cellules humaines.

45. Procédé selon la revendication 44, **caractérisé en ce qu'**on utilise comme cellules humaines des chondrocytes cultivés et multipliés à partir de cellules endogènes.

46. Procédé selon l'une des revendications 42 à 45, **caractérisé en ce que** le support de cellules est relié de façon indétachable au support.

47. Procédé selon l'une des revendications 42 à 46, **caractérisé en ce que** les au moins deux éléments de support de cellules ne sont pas reliés entre eux, directement mais uniquement par l'intermédiaire du support.

48. Procédé selon l'une des revendications 42 à 47, **caractérisé en ce que** les au moins deux éléments de support de cellules sont fabriqués par au moins une incision dans un support de cellules monobloc, de sorte que les au moins deux éléments de support sont séparés par au moins une entaille d'incision.

49. Procédé selon l'une des revendications 42 à 48, **caractérisé en ce que** les au moins deux éléments de support de cellules sont séparés par au moins une fente qui présente au moins une largeur de l'ordre de 0,1 à 0,8 mm.

50. Procédé selon la revendication 49, **caractérisé en ce que** ladite au moins une fente est empilé d'un matériau biocompatible résorbable.

51. Procédé selon l'une des revendications 48 à 50, **caractérisé en ce que** le support de cellules, en vue de la réalisation de la au moins une entaille d'incision ou de la au moins une fente, est incisé au moins jusqu'à la moitié de l'épaisseur de la couche du support de cellules.
